(19) **Europäisches Patentamt / European Patent Office / Office européen des brevets**

(11) **EP 1 456 659 B1**

(12) **EUROPEAN PATENT SPECIFICATION**

(45) Date of publication and mention
of the grant of the patent:
**12.03.2008 Bulletin 2008/11**

(21) Application number: **02793642.6**

(22) Date of filing: **13.12.2002**

(51) Int Cl.:
**G01N 33/543** (2006.01)      **C12Q 1/68** (2006.01)
**B01L 3/00** (2006.01)

(86) International application number:
**PCT/SE2002/002319**

(87) International publication number:
**WO 2003/056337 (10.07.2003 Gazette 2003/28)**

(54) **IMMOBILIZATION OF BINDING AGENTS**

IMMOBILISIERUNG VON BINDUNGSSTOFFEN

IMMOBILISATION D'AGGLUTINANTS

(84) Designated Contracting States:
**AT BE BG CH CY CZ DE DK EE ES FI FR GB GR IE IT LI LU MC NL PT SE SI SK TR**

(30) Priority: **21.12.2001 SE 0104365**
**21.12.2001 US 343343 P**

(43) Date of publication of application:
**15.09.2004 Bulletin 2004/38**

(73) Proprietor: **GE Healthcare Bio-Sciences AB**
**751 84 Uppsala (SE)**

(72) Inventors:
• **LÖFAS, Stefan**
**S-756 46 Uppsala (SE)**
• **SJÖBOM, Hans**
**S-752 59 Uppsala (SE)**

(74) Representative: **Widén, Björn et al**
**GE Healthcare Bio-Sciences AB**
**Patent Department**
**Björkgatan 30**
**751 84 Uppsala (SE)**

(56) References cited:
EP-A2- 0 895 082          WO-A1-98/42730
WO-A2-01/94946          DE-A1- 19 543 232
US-A- 5 474 796          US-A- 5 624 711
US-A- 5 919 626          US-B1- 6 329 209

• ZAKO; TAMOTSU ET AL: 'Monitoring of the refolding process for immobilized firefly luciferase with a biosensor based on surface plasmon resonance' J BIOCHEM vol. 129, no. 1, January 2001, TOKYO, pages 1 - 4 ISSN: 0021-924X

**Description**

**Technical field**

The present invention relates to spatially-addressable immobilization of binding agents on a support surface, and more particularly to a method of preparing an array by spatially immobilizing binding agents at predefined regions on a support surface, an array prepared by the method, the use of the prepared array, a biosensor comprising the array, and an activated solid support surface to which binding agents can be coupled.

**Background of the invention and prior art**

Array technology providing arrays of binding agents, for example ligands, such as oligonucleotides and peptides, on solid supports has become increasingly important in especially the biotechnological and pharmaceutical fields as a tool for performing repetitive assays and screenings of analytes, including gene expression analysis, drug screening, nucleic acid sequencing, mutation analysis, and the like.

Among the techniques used so far for dispensing, or "spotting", the binding agents at discrete positions on the array may be mentioned piezoelectric micropipetting ("ink-jet"), micro-contact printing, capillary stamping, electrical addressing, and inertia-driven ejection of microdrops. For general reviews of so-called microarray technology it may be referred to, for example, Tibtech 1996, 14: 401-407 and Tibtech 1996, 16: 301-306.

An important step in the preparation of microarrays is the immobilization of the binding agents on the support surface. A large variety of methods are known for attaching biomolecules to solid supports, including covalent bonding to the support surface and non-covalent interaction of the biomolecules with the surface.

General descriptions of binding methods are given in, for example, Affinity Techniques. Enzyme Purification: Part B. Methods in Enzymology, Vol. 34, ed. W.B. Jacoby, M. Wilchek, Acad. Press, N.Y. (1974), and Immobilized Biochemicals and Affinity Chromatography, Advances in Experimental Medicine and Biology, vol. 42, ed. R. Dunlop, Plenum Press, N.Y. (1974). Exemplary binding methods are also disclosed in the following publications.

US-A-4,681,870 describes a method for introducing free amino or carboxyl groups onto a silica matrix. These groups may subsequently be covalently linked to, e.g., a protein or other ligand, in the presence of a carbodiimide. Alternatively, a silica matrix may be activated by treatment with cyanogen halide under alkaline conditions. The ligand is covalently attached to the surface upon addition to the activated surface.

US-A-4,282,287 describes a method for modifying a polymer surface through successive application of multiple layers of biotin, avidin and extenders.

US-A-4,762,881 describes a method for attaching a polypeptide to a solid substrate by incorporating a light-sensitive unnatural amino acid group into the polypeptide chain and exposing the product to low-energy ultraviolet light.

Modification of surface characteristics in terms of hydrophilic and hydrophobic properties in the preparation of microarrays has also been described in the prior art.

For example, WO 98/42730 discloses the preparation of a support surface which is hydrophilic in a first state and hydrophobic in a second state, so that the substrate can be used in either aqueous or organic media. The substrate surface contains a plurality of hydrophilic sites which can be readily protected and deprotected. By protecting a fraction of the sites, the surface is provided in a hydrophobic form such that the unprotected sites may participate in organic synthesis processes to be conducted using organic reagents and solvents. Following synthesis, the protected hydrophilic sites are deprotected, regenerating the substrate surface in hydrophilic form for use with aqueous reagents, e.g. in screening and/or separation procedures to be conducted in aqueous media.

US-A-6,127,129 discloses a method of making a biomolecule or cellular array on a metal substrate. An ω-roodiSed alkanethiol monolayer is deposited on the metal substrate to form a hydrophilic surface. The monolayer is then reacted with hydrophobic protecting groups, and the surface is photopatterned to create an array of exposed metal surface areas. Subsequent deposition of ω-modified alkanethiol in the areas of exposed metal substrate yields an array of discrete hydrophilic spots of unprotected ω-modified alkanethiol, to which biomolecules or cells are attached. The protecting groups are then removed from the hydrophobic background surrounding the discrete spots with immobilized biomolecules or cells, and the monolayer is made resistant to non-specific protein binding, e.g. by attaching PEG moieties thereto.

EP-A-895 082 discloses a method for spotting probes on a solid support which comprises reacting maleimido groups on the support surface with thiol group-containing nucleic acid probes added by an ink-jet method. An alternative binding pair is epoxy groups on the surface and amine groups on the nucleic acid probes. Non-specific binding to the surface is prevented by blocking with BSA solution or decomposition of unreacted maleimido groups. A surface containing epoxy groups may be made hydrophilic by opening unreacted epoxy rings with ethanolamine to form hydroxy groups after binding of the nucleic acid probes.

WO 98/55593 discloses the patterning of a solid support with synthetic nucleic acid molecules using, for example, an ink-jet printing delivery technique. The support surface is silanized providing a very hydrophobic surface which allows oligonucleotide probe droplets to form at specific and localized positions on the solid support surface with no cross contamination between probes even at high probe density.

WO 98/39481 discloses methods for covalent, specific immobilization of nucleic acid molecules onto a solid mercaptosilanized hydrophobic surface by a reversible disulfide bond formed by coupling a sulfhydryl- or disulfide-modified nucleic acid molecule to the sulfhydryl groups of the mercaptosilanes. A high probe density without cross contamination may be achieved.

US-A-6,066,44.8 discloses methods for producing patterned multi-array, multi-specific surfaces. The binding domains on the multi-array surface may be hydrophobic or hydrophilic, and the surrounding surface may have the opposite property (hydrophilic or hydrophobic) to that of the binding domains. The use of such a hydrophilic/hydrophobic border aids in confining the produced binding domain to a discrete area on the surface. The hydrophobic and hydrophilic binding domains may be generated by micro-contact printing.

US-A-5,474,796 discloses the preparation of array plates by coating a glass plate with a photoresist substance, exposing to light to obtain a patterned surface of exposed and photoresist-coated surfaces, reacting the exposed surfaces with a hydrophobic reagent, removing the photoresist, and converting the exposed surface areas to hydrophilic binding regions. Alternatively, one starts from a derivatized hydrophilic surface to directly obtain the hydrophilic binding regions in the last step.

US-A-5,985,551 discloses the preparation of an array plate which comprises a support surface having a covalently linked layer of inert siloxane defining an array of 10 to $10^4$ sites per $cm^2$ which do not have the covalently linked layer. Chemical reactant solutions are localized to these sites, which are about 50-2000 microns in diameter, via surface tension.

US-A-6,171,797 discloses a method for making arrays of distinct polymers covalently bonded to the surface of a solid support. At least two distinct polymers, e.g. nucleic acids, are covalently bound to the support surface through a cycloaddition reactive group on the surface capable of reacting with a group present on the polymers in a cycloaddition reaction to produce a covalent linkage between the polymer and the support surface. In many embodiments, the contact angle of the cycloaddition reactive group is sufficient to provide for extremely low drop spreading of fluid deposited on the substrate surface.

WO 01/94946 discloses the preparation of arrays of protein-binding agents. In one embodiment, a gold-surfaced microscope slide is coated with an aminothiol layer that is then functionalized with a group that will bind to an anchor functional group, e.g. a thiol function, of a protein binding agent. After deposition of the protein binding agent(s) to the generally hydrophilic functionalized surface, unreacted maleimides on the surface can be blocked chemically, e.g. with a hydrophilic thiol, to minimize background non-specific binding of proteins. The surface can also be blocked using proteins, such as human serum albumin (HSA).

US 5,624,711 discloses the preparation of supports for solid phase synthesis of oligomer arrays of single compounds. A glass slide or other support is derivatized with an aminoalkylsilane to provide a surface of amine functional groups. This surface is then treated with a mixture of linking molecules (e.g. nitroveratryloxycarbonylaminocaproic acid) and diluent molecules (e.g. protected amino acids) to provide a surface having initiation sites at a preselected density. The linking molecule contributes to the net hydrophobic or hydrophilic nature of the surface and can be selected to improve presentation of the polymer synthesized thereon to certain receptors, proteins or drugs. The diluent molecules can also be selected to impart hydrophobic or hydrophilic properties to the substrate surface. For example, o-t-butylserine as a diluent molecule provides a hydrophobic surface during polymer synthesis but upon treatment with acid, ether cleavage provides a more hydrophilic surface for assays.

US 6,329,209 discloses arrays of different protein capture agents immobilized on discrete patches, e.g. of gold, covered by an organic thinfilm. The surfaces, or border regions, between the patches of protein-capture agents may be covered by a different organic thinfilm with low non-specific binding properties for proteins and other analytes, e.g. a monolayer of hydrophilic chains attached to an ordered hydrophobic monolayer of alkyl chains.

Zako, Tamotsu, et al., J. Biochem. 129, 1-4 (2001) discloses immobilization of a protein (firefly luciferase) on a carboxymethyldextran-coated surface plasmon resonance (SPR) sensor chip by an amine based coupling chemistry. N-ethyl-N'-(3-diethylaminopropyl)-carbodiimide (EDC) and N-hydroxysuccinimide (NHS) are used to activate the surface, and the protein is then contacted with the sensor chip to covalently couple the protein to the surface. Excess NHS-ester groups are blocked by ethanolamine.

Generally, however, the prior art methods for attaching binding agents to surfaces are unsatisfactory in several respects. Deficiences include low reaction efficiences and a general inability to readily permit regional and selective attachment of a plurality of binding agents to the surface. Also, high non-specific binding of analytes to the surface is often observed when performing screenings and assays using ligand-supporting surfaces prepared according to the prior art methods. There is therefore a need of a more efficient and simpler to perform method of binding agent immobilization in the preparation of arrays. Such a method should provide stable attachment of selected binding agents to predefined surface regions, yet the attachment should be strictly restricted to the predefined regions. Also, when performing assays and screenings using the prepared surfaces, non-specific binding of analytes to the surfaces should be low or negligible. The above needs are fulfilled by the present invention which provides further related advantages.

**Summary of the invention**

In brief, the present invention relates to the preparation of arrays of binding agents and is based on the concept of utilizing activation of functional groups to reactive (or more reactive) groups to thereby temporarily change the hydrophilic properties of a functional group-bearing solid support surface. After contacting predefined regions or spots on the surface with liquid medium containing binding agent to form the array of immobilized binding agents, deactivation of the surface brings the surface back to its original hydrophilic state. Such a temporary change of the surface properties may be used in different ways to improve the formation of a desired array.

Temporarily making a generally hydrophilic support surface area hydrophobic by activating functional groups on the hydrophilic surface area (at least at predefined regions or spots thereof) to reactive groups which are hydrophobic, and then contacting the surface area at the predefined regions or spots with one or more aqueous liquids containing binding agent to be immobilized on the surface, may reduce spreading or extension of liquid on the surface and the immobilization of binding agent may be effectively restricted to the predefined regions. After conversion or deactivation of any unreacted reactive groups to more hydrophilic groups, the original generally hydrophilic character of the surface is restored and non-specific binding to the surface is minimized when subsequently using the surface for analysis.

Therefore, in one aspect, the present invention provides a method of preparing an array of discrete localized binding agent-supporting regions on a solid support surface, which method comprises the steps of:

(i) providing a solid support having a surface bearing functional groups at least at a plurality of predefined discrete regions thereon, which surface is in a first surface property state, is hydrophilic,
(ii) reacting functional groups on at least the predefined discrete regions of the solid support surface with an activating agent selected to activate the functional groups to form reactive groups of such a polarity from that any activated surface area is provided in a second state which is hydrophobic having a contact angle with water of at least 40 degrees
(iii) selectively dispensing at each predefined discrete region on the solid support surface a predetermined volume of an aqueous liquid containing a binding agent to couple the binding agent to the reactive groups, the second hydrophobic state substantially reducing spreading of the aqueous liquid when applied to the surface, and
(iv) deactivating unreacted reactive groups on the solid support surface such that the first hydrophilic state of the solid support surface is restored.

In one embodiment, substantially the whole functional group-bearing solid phase surface is subjected to activation conditions. In another embodiment, the activation of functional groups is restricted to the predefined discrete regions on the support surface.

In another aspect, the present invention provides an array of one or more binding agents prepared by the method according to the first aspect.

Other aspects of the present invention provide the use of an array prepared by the method according to the first aspect for studying molecular interactions, and for performing an assay for one or more analytes, respectively.

Still another aspect of the invention relates to a biosensor comprising an array prepared by the method according to the first aspect.

Yet another aspect of the invention relates to an activated solid support surface to which binding agents can be coupled.

**Definitions**

In describing and claiming the present invention, the following terminology will be used in accordance with the definitions set out below.

"Array" as used herein generally relates to a linear or two-dimensional array of discrete regions, each having a finite area, formed on a continuous surface of a solid support and supporting one or more binding agents. Ordered arrays of nucleic acids, proteins, small molecules, cells or other substances on a solid support enable parallel analysis of complex biochemical samples. In a "microarray", the density of discrete regions, or spots, is typically at least $100/cm^2$, and the discrete regions typically have a diameter in the range of about 10-1000 $\mu$m, usually about 10-500 $\mu$m and are separated from other regions in the array by about the same distance.

"Predefined region" as used herein relates to a localized area on the solid support surface. The predefined region may have any desired shape, such as circular, rectangular, elliptical, etc, and is below often referred to as a "spot".

"Solid support" as used herein is meant to comprise any solid (flexible or rigid) substrate onto which it is desired to apply an array of one or more binding agents. The substrate may be biological, non-biological, organic, inorganic or a combination thereof, and may be in the form of particles, strands, precipitates, gels, sheets, tubings, spheres, containers, capillaries, pads, slices, films, plates, slides, etc, having any convenient shape, including disc, sphere, circle, etc. The substrate surface supporting the array may have any two-dimensional configuration and may include, for example steps, ridges, kinks, terraces and the like and may be the surface of a layer of material different from that of the rest of the substrate.

"Functional group" as used herein means a reactive chemical entity that serves to connect a binding agent to the surface. Usually, functional groups need to be activated in order to immobilize a binding agent. The functional groups may be inherently present in the material used for the solid support or they may be provided by treating or coating the support with a suitable material. The functional group may also be introduced by reacting the solid support surface with an appropriate chemical agent.

"Activation" as used herein means a modification of a functional group on the solid support surface to enable coupling of a binding agent to the surface.

"Binding agent" as used herein means any agent that is a member of a specific binding pair, including, for instance polypeptides, such as proteins or fragments thereof; nucleic acids, e.g. oligonucleotides, polynucleotides, and the like; etc. The binding agent is often a ligand.

"Ligand" as used herein means a molecule that has a known or unknown affinity for a given analyte and can be immobilized on a predefined region of the surface. The ligand may be a naturally occurring molecule or one that has been synthesized. The ligand may be used *per se* or as aggregates with another species. Optionally, the ligand may also be a cell.

"Analyte" as used herein is a molecule, typically a macromolecule, such as a polynucleotide or polypeptide, the presence, amount and/or identity of which are to be determined. The analyte is recognized by a particular ligand forming an analyte/ligand pair. Optionally, the ligand may also be a cell.

"Surface property" as used herein relates to the hydrophilic or hydrophobic character of a surface.

"Hydrophobic" as used herein may be defined as water-repelling whereas "hydrophilic" may be defined as water-attracting. With regard to a surface, the terms hydrophobic and hydrophilic may be defined by the contact angle for a droplet of a liquid on a planar solid surface, the contact angle being measured from the plane of the surface, tangent to the surface at the three phase boundary line. A hydrophilic liquid will thus have a low contact angle on a hydrophilic surface, whereas a hydrophobic liquid will have a high contact angle. For example, hydrophobic surfaces typically have contact angles with water in the range of 40 to 110°, while the contact angles with water for hydrophilic surfaces typically are in the range of 1 to 25°. Thus, a support surface is hydrophobic if an aqueous medium droplet applied to the surface does not spread out substantially beyond the area size of the applied droplet, the surface acting to prevent spreading of the droplet applied to the surface by hydrophobic interaction with the droplet.

"Polarity" as used herein refers to the polar/non-polar properties of a chemical group. "Polar" and "non-polar" are related to the terms hydrophilic and hydrophobic. A polar group is hydrophilic, and a non-polar group is hydrophobia. A hydroxy group is an example of a polar group, and an alkyl group is an example of a non-polar group. When it is said, for example, that a group is "less polar", this includes, of course, also that the group may be non-polar, and *vice versa.*

The term "aqueous liquid medium" as used herein refers to a liquid medium containing less than about 50 vol. % of organic solvent, more preferably less than about 10 vol.% organic solvent, and most preferably about 0 vol.% organic solvent.

The terms "non-aqueous liquid medium" and "organic liquid medium" as used herein refer to a liquid medium containing less than about 50 vol.% of water, more preferably less than about 10 vol.% water, and most preferably about 0 vol.% water.

In the specification and the appended claims, the singular forms "a", "an", and "the" are meant to include plural reference unless it is stated otherwise. Also, unless defined otherwise, technical and scientific terms used herein have the same meaning as commonly understood to a person skilled in the art related to the invention.

## Detailed description of the invention

As mentioned above, the present invention relates to the coupling of binding agents, such as ligands, to functional groups on an solid support surface at predefined regions, or spots, thereon to prepare an array of the binding agents on the surface. Usually, it is desired that the array surface exhibits a generally hydrophilic character to prevent nonspecific binding of biomolecular analytes thereto when using the array surface in assays. On the other hand, it is also desired that at least the predefined regions on the array surface have a substantially less hydrophilic character than the rest of the array surface, and preferably a generally hydrophobic character, to prevent extensive spreading of droplets of aqueous liquid containing the binding agents when contacting the surface therewith to couple the binding agents to the surface.

The invention favourably makes use of activation of the functional groups to reactive groups with selected activating agents to provide hydrophobic surface to be contacted with the binding agent-containing liquid droplets. In this way, a hydrophilic surface may temporarily be made hydrophobic and thereby satisfy both the above needs, i.e. exhibit hydrophobic character for the coupling of the binding agents to substantially reduce or eliminate the spreading of the binding agents on the surface, and after the coupling provide the desired hydrophilic character of the noncoupled areas to reduce or eliminate non-specific binding. Due to the low spreading on the activated surface areas, the binding agent-supporting regions may be confined and well defined and, if desired, permit a high density of binding agent-regions or spots, despite the use of a water-based binding agent-containing liquid.

In one method embodiment of the invention, functional groups on the surface are converted to reactive groups by selective activation of the functional group on predefined spots on the surface. The activated reactive groups are then used to immobilize binding agents, e.g. ligands, at the predefined spots by dispensing a predetermined liquid volume containing binding agent at each spot. The procedure may be repeated at different sites on the surface so as to provide a surface prepared with a plurality of spots on the surface which contain the same or different binding agents. If the binding agents are ligands having a particular affinity for one or more analytes, screenings and assays can be conducted in the regions of the surface containing the ligands, as will be further described below. In some embodiments, a first binding agent bound to the solid support surface serves as a capturer for a second binding agent which may be a ligand. After preparation of the array, the surface may be dried and stored for subsequent use, if desired.

In an alternative method embodiment, functional groups on the entire surface are activated rather than only at the predefined spots, and the binding agents are then selectively bound to the predefined spots.

The selective dispensing of the predetermined liquid volumes to the predefined spots may preferably be performed by a deposit device as will be described further below.

The use of activation according to the present invention thus enables (preferably covalent) coupling of the binding agents and at the same time provides the desired temporary surface property to the immobilization regions or spots, i.e. making a hydrophilic surface hydrophobic in order to prevent aqueous solutions of binding agents from spreading when contacted with the activated regions. This in turn aids in concentrating and confining the immobilization of binding agents to the predefined regions. An exemplary schematic illustration of the method of the invention is given below.

The activation (A) of functional groups (F) on a surface (S) of a solid substrate may generally be illustrated by the following equation:

$$\text{S-F} + \text{A} \rightarrow \text{S-F-A}$$

Predefined regions, or spots, ($S_i$) on the surface may be activated for ultimate immobilization of ligands at the predefined regions by selectively dispensing (depositing) an activation mixture at the predefined regions to convert functional groups to activated groups. The process is illustrated by the equation:

$$\text{S}_i\text{-F} + \text{A} \rightarrow \text{S}_i\text{-F-A}$$

Immobilization of a ligand ($L_i$) on a predefined region of the surface is illustrated by the equation:

$$\text{S}_i\text{-F-A} + \text{L}_i \rightarrow \text{S}_i\text{-F-L}_i$$

Immobilization of a different ligand ($L_j$) on a different region ($S_j$) of the surface may be shown by the equation:

$$\text{S}_j\text{-F-A} + \text{L}_j \rightarrow \text{S}_j\text{-F-L}_j$$

Repetition of the above steps on different regions of the surface will produce a matrix of ligands immobilized on the substrate surface. Such a matrix may have any desired pattern of ligands.

An immobilized ligand on a surface will have a specific binding affinity for a particular analyte (An). An example of a direct assay at a predefined region of the surface for the presence of the analyte (An') in a liquid medium is illustrated by the equation:

$$S_i\text{-}F\text{-}L_i + An' \rightarrow S_i\text{-}F\text{-}L_i\text{-}An'$$

The original hydrophilic character of the substrate surface bearing the functional group may be provided by (i) the functional group only, (ii) both the functional group and the substrate surface material, or (iii) the substrate surface material only. In the first-mentioned case (i), the functional group should be relatively hydrophilic, whereas in case (ii) the functional group may also have a moderate or low hydrophilicity. In case (iii), the functional group may even be slightly hydrophobic as long as the substrate surface material otherwise has a sufficient hydrophilicity to make the surface generally hydrophilic.

A wide variety of activatable functional groups well-known to the skilled person may be used in the invention. Examples of such hydrophilic groups are hydroxy, carboxy, carbonyl, formyl, amino, and mercapto groups, just to mention a few.

Methods for activating the functional groups are readily apparent to the skilled person and may be selected from a wide variety of methods.

The activating agent is selected to provide an activated surface, or activated surface regions, that is/are hydrophobic having a contact angle (as defined above) in the range of from about 40 to about 100°, and especially from about 60 to 100°.

Activating agents and methods that may be used depend, of course, on the functional group to be activated and on the desired reactive group to be obtained by the activation. Exemplary functional group/activating agent combinations include those introducing nitro- and dinitrophenyl esters, tosylates, mesylates, triflates and disulfides. For example, a hydroxy group may be reacted to activated ester with disuccinic carbonate, or to epoxide with a diepoxide. A carboxy group may be activated to to dinitrophenyl ester by reaction with dinitrophenol. A thiol (mercapto) group may be activated to a disulfide group by reaction with dipyridyldisulfides.

To immobilize the binding agents on the surface, the activated functional groups, which, as mentioned above, may be present on the predefined regions only, or alternatively on the entire surface, are reacted selectively at the predefined regions with the binding agent or agents. The necessary reaction conditions, including time, temperature, pH, solvent(s), additives, etc will depend on *inter alia* the particular species used and appropriate conditions for each particular situation will readily be apparent to the skilled person.

The liquid medium containing the binding agent, usually a solution thereof, may be applied to the respective predefined region by selectively depositing a predetermined volume of the liquid on the surface of the solid support. Such deposition may be performed manually, e.g. via a pipette, or through the use of an automated machine or device. A wide variety of devices for dispensing or depositing aqueous solutions onto precise locations of a support surface, so-called "arrayers", are known to the skilled person and may be employed in the present invention. Such devices include "ink-jet" printing devices (including piezoelectric and thermal devices), "pin-and-ring" devices, devices for micro-contact printing, devices for capillary stamping, and the like. Reference may be made to, for example, US-A-4,877, 745, US-A-5,338,688, US-A-5,474,996, US-A-5,658,802, US-A-6,165,417, WO 00/56455, WO 00/56433, WO 00/56442, and WO 98/51999. Commercial arrayers are available from a number of vendors, including e.g. Packard, Biorobotics, Affymetrix, Techan, Cartesian Technologies, Gene Machines, Molecular Dynamics, and HSG-IMIT.

The predetermined liquid volume to be dispensed onto each individual surface region or spot depends on *inter alia* the spot size, the density of the functional group, the binding agent and its concentration, the deposit device, etc, and suitable liquid volumes in each particular situation may readily be selected by the skilled person.

When a hydrophilic surface is temporarily made hydrophobic the hydrophobic character of the surface regions supporting the activated functional groups will provide for extremely low spreading of an aqueous fluid deposited on the surface. The resulting spots of immobilized binding agent on the surface may therefore be rather small and usually have a diameter below about 1 mm, particularly below about 500 $\mu$m, and especially do not exceed about 200 $\mu$m. On the other hand, the spot diameter is preferably not smaller than about 1 $\mu$m, more preferably not smaller than about 5 $\mu$m, and especially not smaller than about 10 $\mu$m.

Depending on the intended application of the array to be prepared, the same or different binding agents, or groups of different binding agents, may be bound to the plurality of predefined regions on the solid support surface.

After immobilizing the binding agent or agents to the surface, any residual reactive groups may be inactivated by treatment with an appropriate wash solution to restore the generally hydrophilic character of the surface. Such inactivating agents are well known to the skilled person and may readily be selected depending on the particular reactive groups on the surface.

The solid support is preferably a rigid structure and may comprise a substrate having a surface layer of a different material. Exemplary substrate materials are Langmuir Blodgett films, functionalized glass, Si, Ge, GaAs, GaP, $SiO_2$, $SiN_4$, modified silicon, and a wide variety of polymers, such as (poly)tetrafluoroethylene, (poly)vinylidenedifluoride, or combinations thereof. A preferred substrate material for many applications is flat glass.

The surface of the solid support may be composed of a variety of materials, for example, polymers, plastics, resins, polysaccharides, silica or silica-based materials, carbon, metals, inorganic glasses, membranes, etc, provided that the surface may support functional groups. A suitable surface is a metal film, e.g. gold, silver, or aluminium, preferably gold.

The solid support surface may be provided with a layer of a polymer. In such a case the polymers will carry the functional groups to be activated. The polymer may be selected from any suitable class of compounds, for example, polyethylene glycols, polyethylene imides, polysaccharides, polypeptides, or polynucleotides, just to mention a few. Attachment of the polymers to the support surface may be effected by a variety of methods which are readily apparent to a person skilled in the art. For example, polymers bearing trichlorosilyl or trisalkoxy groups may be reacted with hydroxyl groups on the substrate surface to form siloxane bonds. Attachment to a gold or silver surface may take place via thiol groups on the polymer. Alternatively, the polymer may be attached via an intermediate species, such as a self-assembled monolayer of alkanethiols. The type of polymers selected, and the method selected for attaching the polymers to the surface, will thus depend on the polymer having suitable reactivity for being attached to the substrate surface, and on the properties of the polymers regarding non-specific adsorption to, especially, proteins.

The functional groups may be present on the polymer or may be added to the polymer by the addition of single or multiple functional groups. Such functional groups are preferably heterobifunctional, having one end adapted to react with reactive groups on the polymer and the other end adapted to react with the activating agent. Methods for attaching the functional groups are readily apparent to the person skilled in the art and may be selected from a wide variety of groups. Exemplary methods are the amidation of amine groups on the polymer with succinic acid, and the reaction of hydroxy groups on the polymer with bromoacetic acid.

As mentioned above, the binding agent is usually a ligand, i.e. a molecule capable of recognizing a particular analyte in solution, Examples of ligands include, without any limitation thereto, agonists and antagonists for cell membranes, toxins and venoms, viral epitopes, antigenic determinants, hormones and hormone receptors, steroids, peptides, enzymes, substrates, cofactors, drugs, lectins, sugars, oligonucleotides, oligosaccharides, proteins, glycoproteins, cells, cellular membranes, organelles, cellular receptors, vitamins, and immunoglobulins, *e.g.* monoclonal and polyclonal antibodies.

Among ligands of particular interest may be mentioned those mediating a biological function on binding with a particular analyte(s). Suitable ligands are often relatively small single molecules, such as cofactors, which exhibit specific binding properties. Typically, ligands will have a size larger than about 100 D, especially larger than about 1 kD. Other examples of (currently) interesting ligands include, without any restriction thereto, the common class of receptors associated with the surface membrane of cells and include, for instance, the immunologically important receptors of B-cells, T-cells, macrophages and the like.

Analytes that may be assayed for include, without any restriction thereto, agonists and antagonists for cell membrane receptors, toxins and venoms, viral epitopes, hormones (e.g. opiates, steroids, etc), hormone receptors, peptides, enzymes, enzyme substrates, cofactors, drugs, lectins, sugars, oligonucleotides, oligosaccharides, proteins, and monoclonal antibodies.

An array surface prepared according to the method of the invention described above may be used for screening analytes having affinity for the immobilized ligands. Such a screening assay may be performed by contacting a solution containing an analyte (or analytes) with the surface for a suitable period of time. By determining those regions on the surface to which the analyte (or analytes) associates when the analyte is contacted with the surface, the ligands having affinity for the analyte may be identified.

Methods for detecting the presence of bound analyte(s) on the surface may be chosen from a wide variety of detection techniques, including, for example, marker-based techniques, where the analyte(s) or an analyte specific reagent is labelled, e.g. with a radiolabel, a chromophore, fluorophore, chemiluminescent moiety or a transition metal.

For many applications, the assays are performed with a biosensor. Biosensors may be based on a variety of detection methods. Typically such methods include, but are not limited to, mass detection methods, such as piezoelectric, optical, thermo-optical and surface acoustic wave (SAW) device methods, and electrochemical methods, such as potentiometric, conductometric, amperometric and capacitance methods. With regard to optical detection methods, representative methods include those that detect mass surface concentration, such as reflection-optical methods, including both internal and external reflection methods, angle, wavelength or phase resolved, for example ellipsometry and evanescent wave spectroscopy (EWS), the latter including surface plasmon resonance (SPR) spectroscopy, Brewster angle refractometry, critical angle refractometry, frustrated total reflection (FTR), evanescent wave ellipsometry, scattered total internal reflection (STIR), optical wave guide sensors, evanescent wave-based imaging such as critical angle resolved imaging, Brewster angle resolved imaging, SPR angle resolved imaging, and the like. Further, photometric methods based on, for example, evanescent fluorescence (TTRF) and phosphorescence may also be employed, as well as waveguide interferometers.

One exemplary type of SPR-based biosensors is sold by Biacore AB (Uppsala, Sweden) under the trade name BIACORE® (hereinafter referred to as "the BIACORE instrument'). These biosensors utilize a SPR based mass-sensing technique to provide a "real-time" binding interaction analysis between a surface bound ligand and an analyte of interest. A typical output from the BIACORE instrument is a "sonsorgram," which is a plot of response (measured in "resonance units" or "RU") as a function of time. An increase of 1000 RU corresponds to an increase of mass on the sensor surface of approximately 1 ng/mm$^2$.

A detailed discussion of the technical aspects of the BIACORE instrument and the phenomenon of SPR may be found in U.S. Patent No: 5,313,264. More detailed information on matrix coatings for biosensor sensing surfaces is given in, for example, U.S. Patents Nos. 5,242,828 and 5,436,161. In addition, a detailed discussion of the technical aspects of the biosensor chips used in connection with the BIACORE instrument may be found in U.S. Patent No. 5,492,840.

In the following Examples, various aspects of the present invention are disclosed more specifically for purposes of illustration and not limitation.

### EXAMPLE 1 (Comparative)

### Activation of a sensor surface (entire surface)

A sensor chip CM5 (a biosensor chip having a gold surface with a covalently linked carboxymethyl-modified dextran polymer hydrogel; Biacore AB, Uppsala, Sweden) was rinsed with deionized water and dried by a stream of nitrogen. 50 $\mu$l of a fresh solution of 0.2 M N-ethyl-N'-(dimethylaminopropyl)carbodiimide (EDC) and 0.05 M N-hydroxysuccininude (NHS) were added to the surface. The solution covered the entire surface as a thin film of liquid. Immidiately after adding the EDC/NHS solution, the sensor chip was put in a sealed box with high humidity to prevent evaporation from the surface. After 20 minutes, the sensor chip was taken out of the sealed box, rinsed with deionized water and dried by a stream of nitrogen. The dried sensor chip was stored at dry conditions for up to 8 hours before immobilization.

The contact angle of the chip surface was measured before and after NHS/EDC activation with a contact angle measuring device (FTA200, First Ten Angstrom, U.S.A.). Before activation, the contact angle of the sensor chip CM5 surface was 10°, and after activation 24°.

### EXAMPLE 2 (Comparative)

### Activation of a sensor surface (predefined regions)

A sensor chip CM5 (Biacore AB) was rinsed with ionized water, dried by a stream of nitrogen and mounted on a holder. 100 x 100 pl of a fresh solution of 0.2 M EDC and 0.05 M NHS were added, using an ink-jet device (AutoDrop-Micropipette AD-K 501, Microdrop, Germany) to the surface. The EDC/NHS solution was located as a 10 x 10 matrix, with a pitch of 200 $\mu$m. The diameter of the spots was approximately 100 $\mu$m. Immediately after adding the EDC/NHS solution, the sensor chip was put in a sealed box with high humidity to prevent evaporation from the surface. After 20 minutes, the sensor chip was taken out of the sealed box, rinsed with deionized water and dried by a stream of nitrogen. The dried sensor chip was stored under dry conditions for up to 4 hours before immobilization was performed.

### EXAMPLE 3 (comparative)

### Immobilization of antibodies to an activated surface

A sensor chip CM5 (Biacore AB) that had been activated according to Example 1 or Example 2 above was mounted to a holder. 100 pl of a solution of 10 mg/ml solution of anti-myoglobin antibody in 10 mM sodium acetate, pH 5.0, were added to the surface using the ink-jet device in Example 2. The diameter of the spots was approximately 100 $\mu$m. Immediately after adding the antibody solution, the sensor chip was put in a sealed box with high humidity to prevent evaporation from the surface. After 24 hours, the sensor chip was taken out of the sealed box, rinsed with ionized water and dried by a stream of nitrogen. When comparing the response for the immobilized spots with the response for non-immobilized regions on the sensor chip, a difference of about 5000 RU was obtained, corresponding to about 5 ng/mm$^2$ of immobilized antibody.

### EXAMPLE 4

### Activation of a sensor surface with different activating agents

A sensor chip CM5 (Biacore AB) was activated as described in Example 1 but using other activating agents than NHS/EDC, and the contact angles of the prepared surfaces were measured with the measuring device used in Example 1. The contact angle of the sensor chip CM5 surface before activation was 10°, and the contact angles obtained after activation with the different activating agents are given in Table 1 below.

**TABLE 1**

| Activating agent | Contact angle |
|---|---|
| 3-Hydroxy-3,4-dihydrobenzotriazole (comparative example) | 38° |
| 1-Hydroxy-7-azabenzotriazole | 43° |
| 1-Hydroxybenzotriazole (comparative example) | 36° |
| Sodium phenol-4-sulfonate (comparative example) | 19° |
| Sodium 2-nitrophenol-4-sulfonate | 45° |

It is to be understood that the invention is not limited to the particular embodiments of the invention described above, but the scope of the invention will be established by the appended claims.


**Claims**

1.  A method of preparing an array of discrete localized binding agent-supporting regions on a solid support surface, which method comprises the steps of

    (i) providing a solid support having a surface bearing functional groups at least at a plurality of predefined discrete regions thereon, which surface in a first state is hydrophilic,
    (ii) reacting functional groups on at least the predefined discrete regions of the solid support surface with an activating agent selected to activate the functional groups to form reactive groups of such a polarity that any activated surface area is provided in a second state which is hydrophobic having a contact angle with water of at least 40 degrees,
    (iii) selectively dispensing at each predefined discrete region on the solid support surface a predetermined volume of an aqueous liquid medium containing a binding agent to couple the binding agent to the reactive groups, the second hydrophobic state substantially reducing spreading of the aqueous liquid medium when applied to the surface, and
    (iv) deactivating unreacted reactive groups on the solid support surface such that the first hydrophilic state of the solid support surface is restored.

2.  The method according to claim 1, wherein substantially the whole solid support surface bears the functional groups.

3.  The method according to claim 1 or 2, wherein the first hydrophilic state of the solid support surface is provided at least partially by the functional groups.

4.  The method according to claim 1, 2 or 3, wherein in step (ii) of claim 1 only the predefined discrete regions on the solid support surface are subjected to activation.

5.  The method according to claim 1, 2 or 3, wherein in step (ii) of claim 1 substantially the whole functional group-bearing solid support surface is subjected to activation.

6.  The method according to any one of claims 1 to 5, wherein the binding agent is covalently coupled to the activated functional groups.

7.  The method according to any one of claims 1 to 6, wherein each solid support surface area supporting functional groups before activation thereof has a contact angle less than 20°, and each activated surface area has a contact angle higher than 50°.

8.  The method according to any one of claims 1 to 7, wherein the functional groups are selected from carboxy, hydroxy, formyl, amino and mercapto groups.

9. The method according to any one of claims 1 to 8, wherein the activating agent is capable of activating the functional groups to reactive groups selected from nitrophenyl ester, dinitrophenyl ester, tosylate, mesylate, triflate and disulfide groups.

10. The method according to any one of claims 1 to 9, wherein the predefined discrete regions are spots having a diameter in the range of from 10 to 1000 $\mu$m.

11. The method according to any one of claims 1 to 10, wherein the solid support comprises a gold film.

12. The method according to any one of claims 1 to 11, wherein the solid support surface comprises extending polymer chains which bear a functional group.

13. The method according to claim 12, wherein the polymer forms a hydrogel.

14. The method according to any one of claims 1 to 13, wherein the liquid medium containing a binding agent is deposited onto the predefined regions by a deposit device.

15. The method according to claim 14, wherein the deposit device is based on ink-jet printing, micro-contact printing, capillary stamping, or inertia-driven ejection of microdrops.

16. A solid support surface having an array of one or more binding agents prepared by the method according to any one of claims 1 to 15.

17. The solid support surface according to claim 16, which is a sensor surface.

18. A biosensor comprising a sensor surface according to claim 17.

19. The biosensor according to claim 18, which uses a label-free detection technique.

20. The biosensor according to claim 19, wherein the detection technique comprises mass-sensing.

21. The biosensor according to claim 20, wherein the detection technique comprises evanescent wave sensing, preferably surface plasmon resonance (SPR).

22. Use of the solid support surface according to claim 16 or 17 for studying interaction of one or more molecular species with one or more immobilized binding agents.

23. Use of the solid support surface according to claim 16 or 17 for performing an assay for one or more analytes.

24. A solid support with a surface having a plurality of predefined discrete regions thereon, wherein each discrete region bears functional groups which have been activated to be capable of coupling a binding agent thereto, wherein each surface area outside the predefined regions is hydrophilic, and wherein the activated functional groups (i) have a polarity such that the predefined regions are hydrophobic having a contact angle with water of at least 40 degrees, and (ii) are capable of being deactivated to obtain a polarity which makes the predefined regions hydrophilic.

**Patentansprüche**

1. Verfahren zum Herstellen eines Arrays getrennter lokalisierter Bindungsmitteltragender Bereiche auf einer Oberfläche eines festen Trägers, wobei das Verfahren die Schritte umfasst:

(i) Bereitstellen eines festen Trägers mit einer Oberfläche, die funktionelle Gruppen an mindestens einer Mehrzahl vordefinierter separater Bereiche darauf trägt, wobei die Oberfläche in einem ersten Zustand hydrophil ist,
(ii) Umsetzen funktioneller Gruppen auf mindestens den vordefinierten getrennten Regionen der Oberfläche des festen Trägers mit einem Aktivierungsmittel, das ausgewählt ist, um die funktionellen Gruppen zu aktivieren, um reaktive Gruppen einer derartigen Polarität zu bilden, dass eine beliebige aktivierte Oberfläche bereitgestellt wird in einem zweiten Zustand, der hydrophob ist, mit einem Kontaktwinkel mit Wasser von mindestens 40°,
(iii) selektiv Abgeben eines vorbestimmten Volumens eines wässrigen flüssigen, ein Bindungsmittel enthaltenden Mediums an jedem vordefinierten getrennten Bereich auf der Oberfläche des festen Trägers, um das Bindungsmittel an die reaktiven Gruppen zu binden, wobei der zweite hydrophobe Zustand im wesentlichen das Verteilen des wässrigen flüssigen Mediums reduziert, wenn es auf der Oberfläche aufgebracht wird, und (iv) Deaktivieren nicht umgesetzter reaktiver Gruppen auf der Oberfläche des festen Trägers derart, dass der erste hydrophile Zustand der Oberfläche des festen Trägers wiederhergestellt wird.

2. Verfahren nach Anspruch 1, wobei im wesentlichen die gesamte Oberfläche des festen Trägers die funktionellen Gruppen trägt.

3. Verfahren nach Anspruch 1 oder 2, wobei der erste hydrophile Zustand der Oberfläche des festen Trägers zumindest teilweise durch die funktionellen Gruppen bereitgestellt wird.

4. Verfahren nach Anspruch 1, 2 oder 3, wobei in Schritt (ii) des Anspruchs 1 nur die vordefinierten getrennten Bereiche der Oberfläche des festen Trägers einer Aktivierung unterzogen werden.

5. Verfahren nach Anspruch 1, 2 oder 3, wobei in Schritt (ii) des Anspruchs 1 im wesentlichen die gesamte, funktionelle Gruppen tragende Oberfläche des festen Trägers einer Aktivierung unterzogen wird.

6. Verfahren nach einem der Ansprüche 1 bis 5, wobei das Bindungsmittel kovalent an die aktivierten funktionellen Gruppen gekoppelt wird.

7. Verfahren nach einem der Ansprüche 1 bis 6, wobei jede Oberfläche des festen Trägers, die funktionelle Gruppen trägt, vor deren Aktivierung einen Kontaktwinkel von weniger als 20° besitzt, und jede aktivierte Oberfläche einen Kontaktwinkel von höher als 50° besitzt.

8. Verfahren nach einem der Ansprüche 1 bis 7, wobei die funktionellen Gruppen ausgewählt sind aus Carboxy-, Hydroxy-, Formyl-, Amino- und MercaptoGruppen.

9. Verfahren nach einem der Ansprüche 1 bis 8, wobei das Aktivierungsmittel fähig ist, die funktionellen Gruppen zu reaktiven Gruppen zu aktivieren, die ausgewählt sind aus Nitrophenylester-, Dinitrophenylester-, Tosylat-, Mesylat-, Triflat- und Disulfidgruppen.

10. Verfahren nach einem der Ansprüche 1 bis 9, wobei die vordefinierten getrennten Bereiche Punkte sind, mit einem Durchmesser im Bereich von 10 bis 1000 $\mu$m.

11. Verfahren nach einem der Ansprüche 1 bis 10, wobei der feste Träger einen Goldfilm umfasst.

12. Verfahren nach einem der Ansprüche 1 bis 11, wobei die Oberfläche des festen Trägers verlängernde Polymerketten umfasst, die eine funktionelle Gruppe tragen.

13. Verfahren nach Anspruch 12, wobei das Polymer ein Hydrogel bildet.

14. Verfahren nach einem der Ansprüche 1 bis 13, wobei das flüssige Medium, das ein Bindungsmittel enthält, auf den vordefinierten Bereichen durch eine Aufbringungsvorrichtung aufgebracht wird.

15. Verfahren nach Anspruch 14, wobei die Aufbringungsvorrichtung basiert auf Tintenstrahldrucken, Mikrokontaktdrucken, Kapillarstempeln oder eine durch Schwerkraft angetriebene Ausstrahlung von Mikrotropfen.

16. Oberfläche eines festen Trägers mit einem Array von einem oder mehr Bindungsmitteln, hergestellt durch das Verfahren nach einem der Ansprüche 1 bis 15.

17. Oberfläche eines festen Trägers nach Anspruch 16, die eine Sensoroberfläche ist.

18. Biosensor, umfassend eine Sensoroberfläche nach Anspruch 17.

19. Biosensor nach Anspruch 18, der eine Marker-freie Detektionstechnik nutzt.

20. Biosensor nach Anspruch 19, wobei die Detektionstechnik eine Massendetektion umfasst.

**21.** Biosensor nach Anspruch 20, wobei die Detektionstechnik die Detektion von evaneszenten Wellen, bevorzugt Oberflächenplasmonenresonanz (SPR) umfasst.

**22.** Verwendung der Oberfläche eines festen Trägers nach Anspruch 16 oder 17 zum Studieren der Wechselwirkung von einer oder mehreren molekularen Spezies mit einem oder mehreren immobilisierten Bindungsmitteln.

**23.** Verwendung der Oberfläche des festen Trägers nach Anspruch 16 oder 17 zum Ausführen eines Assays für einen oder mehrere Analyten.

**24.** Fester Träger mit einer Oberfläche mit einer Mehrzahl vordefinierter getrennter Bereiche darauf, wobei jeder getrennte Bereich funktionelle Gruppen trägt, die aktiviert worden sind, um fähig zu sein, ein Bindungsmittel daran zu koppeln, wobei jede Oberfläche außerhalb der vordefinierten Bereiche hydrophil ist, und wobei die aktivierten funktionellen Gruppen (i) eine Polarität derart besitzen, dass die vordefinierten Bereiche hydrophob sind mit einem Kontaktwinkel mit Wasser von mindestens 40°, und (ii) fähig sind, deaktiviert zu werden, um eine Polarität zu erhalten, die die vordefinierten Bereiche hydrophil macht.

**Revendications**

**1.** Procédé de préparation d'une matrice de zones support d'agent de liaison localisées discrètes sur une surface support solide, lequel procédé comprend les étapes de :

(i) production d'un support solide comportant une surface support de groupes fonctionnels au moins au niveau d'une pluralité de zones discrètes prédéfinies sur celui-ci, laquelle surface, dans un premier état, est hydrophile,
(ii) mise en réaction de groupes fonctionnels sur au moins les zones discrètes prédéfinies de la surface support solide avec un agent d'activation sélectionné afin d'activer les groupes fonctionnels de manière à former des groupes réactifs d'une telle polarité qu'une section de surface activée est placée dans un second état qui est hydrophobe, présentant un angle de contact avec l'eau d'au moins 40 degrés,
(iii) distribution sélective au niveau de chaque zone discrète prédéfinie sur la surface support solide d'un volume prédéterminé d'un milieu liquide aqueux contenant un agent de liaison afin de coupler l'agent de liaison aux groupes réactifs, le second état hydrophobe réduisant sensiblement l'épandage du milieu liquide aqueux lorsqu'il est appliqué sur la surface, et
(iv) désactivation des groupes réactifs qui n'ont pas réagi sur la surface support solide de telle sorte que le premier état hydrophile de la surface support solide est rétabli.

**2.** Procédé selon la revendication 1, dans lequel sensiblement la totalité de la surface support solide supporte les groupes fonctionnels.

**3.** Procédé selon la revendication 1 ou 2, dan lequel le premier état hydrophile de la surface support solide est assuré au moins partiellement par les groupes fonctionnels.

**4.** Procédé selon la revendication 1, 2 ou 3, dans lequel, à l'étape (ii) de la revendication 1, seules les zones discrètes prédéfinies sur la surface support solide sont soumises à une activation.

**5.** Procédé selon la revendication 1, 2 ou 3, dans lequel, à l'étape (ii) de la revendication 1, sensiblement la totalité de la surface support solide support de groupe fonctionnel est soumise à une activation.

**6.** Procédé selon l'une quelconque des revendications 1 à 5, dans lequel l'agent de liaison est couplé de manière covalente aux groupes fonctionnels activés.

**7.** Procédé selon l'une quelconque des revendications 1 à 6, dans lequel chaque zone de surface support solide supportant des groupes fonctionnels avant leur activation présente un angle de contact inférieur à 20 degrés, et chaque zone de surface activée présente un angle de contact supérieur à 50 degrés.

**8.** Procédé selon l'une quelconque des revendications 1 à 7, dans lequel les groupes fonctionnels sont sélectionnés à partir des groupes carboxy, hydroxy, formyle, amino et mercapto.

**9.** Procédé selon l'une quelconque des revendications 1 à 8, dans lequel l'agent d'activation peut activer les groupes fonctionnels en groupes réactifs sélectionnés à partir des groupes ester de nitrophényle, ester dinitrophényle, tosylate, mésylate, triflate et bisulfure.

10. Procédé selon l'une quelconque des revendications 1 à 9, dans lequel les zones discrètes prédéfinies sont des points présentant un diamètre dans la plage de 10 à 1000 $\mu$m.

11. Procédé selon l'une quelconque des revendications 1 à 10, dans lequel le support solide comprend un film d'or.

12. Procédé selon l'une quelconque des revendications 1 à 11, dans lequel la surface support solide comprend des chaînes polymères d'extension qui supportent un groupe fonctionnel.

13. Procédé selon la revendication 12, dans lequel le polymère forme un hydrogel.

14. Procédé selon l'une quelconque des revendications 1 à 13, dans lequel le milieu liquide contenant un agent de liaison est déposé sur les zones prédéfinies par un dispositif de revêtement.

15. Procédé selon la revendication 14, dans lequel le dispositif de revêtement est basé sur l'impression à jet d'encre, l'impression par microcontact, le compostage capillaire ou l'éjection entraînée par inertie de microgouttelettes.

16. Surface support solide comportant une matrice d'un ou plusieurs agents de liaison préparée par le procédé selon l'une quelconque des revendications 1 à 15.

17. Surface support solide selon la revendication 16, qui est une surface de capteur.

18. Biocapteur comprenant une surface de capteur selon la revendication 17.

19. Biocapteur selon la revendication 18, qui utilise un procédé de détection sans marqueur.

20. Biocapteur selon la revendication 19, dans lequel le procédé de détection comprend la détection de masse.

21. Biocapteur selon la revendication 20, dans lequel le procédé de détection comprend la détection d'ondes évanescentes, de préférence, la résonance de plasmon de surface (SPR).

22. Utilisation d'une surface support solide selon la revendication 16 ou 17 pour l'étude d'interaction d'une ou plusieurs espèces moléculaires avec un ou plusieurs agents de liaison immobilisés.

23. Utilisation d'une surface support solide selon la revendication 16 ou 17, afin d'exécuter un dosage d'un ou plusieurs analytes.

24. Support solide avec une surface présentant une pluralité de zones discrètes prédéfinies au-dessus, dans lequel chaque zone discrète supporte des groupes fonctionnels qui ont été activés de manière à pouvoir assurer le couplage d'un agent de liaison à celle-ci, dans lequel chaque section de surface à l'extérieur de zones prédéfinies est hydrophile, et dans lequel les groupes fonctionnels activés (i) présentent une polarité de telle sorte que les zones prédéfinies sont hydrophobes, présentant un angle de contact avec l'eau d'au moins 40 degrés, et (ii) peuvent être désactivés afin d'obtenir une polarité qui rend les zones prédéfinies hydrophiles.

EP 1 456 659 B1

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 4681870 A
- US 4282287 A
- US 4762881 A
- WO 9842730 A
- US 6127129 A
- EP 895082 A
- WO 9855593 A
- WO 9839481 A
- US 606644 A
- US 5474796 A
- US 5985551 A
- US 6171797 A
- WO 0194946 A
- US 5624711 A
- US 6329209 B
- US 4877745 A
- US 5338688 A
- US 5474996 A
- US 5658802 A
- US 6165417 A
- WO 0056455 A
- WO 0056433 A
- WO 0056442 A
- WO 9851999 A
- US 5313264 A
- US 5242828 A
- US 5436161 A
- US 5492840 A

**Non-patent literature cited in the description**

- *Tibtech,* 1996, vol. 14, 401-407
- *Tibtech,* 1996, vol. 16, 301-306
- Affinity Techniques. Enzyme Purification: Part B. Methods in Enzymology. Acad. Press, 1974, vol. 34
- Immobilized Biochemicals and Affinity Chromatography. Advances in Experimental Medicine and Biology. Plenum Press, 1974, vol. 42
- **ZAKO, TAMOTSU et al.** *J. Biochem.,* 2001, vol. 129, 1-4

15